# EUROPEAN PATENT APPLICATION

(11) **EP 4 588 422 A1**
(43) Date of publication of application: **23.07.2025**
(21) Application number: 23865623.5
(22) Date of filing: 15.09.2023
(51) Int. Cl.: A61B 1/018

(54) **ENDOSCOPE TREATMENT TOOL AND ENDOSCOPE TREATMENT SYSTEM**

(30) Priority: 16.09.2022 US 202263375987 P
(71) Applicant: Olympus Medical Systems Corp., Tokyo 192-8507 (JP)
(72) Inventor: UEMICHI Katsuji, Hachioji-shi, Tokyo 192-8507 (JP); SATO Keiichi, Hachioji-shi, Tokyo 192-8507 (JP); OZAWA Keita, Hachioji-shi, Tokyo 192-8507 (JP); SHOJI Keita, Hachioji-shi, Tokyo 192-8507 (JP); YANUMA Yutaka, Hachioji-shi, Tokyo 192-8507 (JP); SAKAI Ryosuke, Hachioji-shi, Tokyo 192-8507 (JP); OTA Hiroyuki, Hachioji-shi, Tokyo 192-8507 (JP)
(74) Representative: Schicker, Silvia
(86) International application number: PCT/JP2023/033769
(87) International publication number: WO 2024/058274

(57) **Abstract**

An endoscopic treatment tool includes a sheath that is an elongated member having a lumen configured to extend in a longitudinal direction, a distal end treatment part provided at a distal end portion of the sheath, an operation part provided at a proximal end portion of the sheath, and a wire inserted through the lumen, mounted on the distal end treatment part, and supported by the operation part so as to advance and retract in the longitudinal direction, wherein the operation part has a locking mechanism that restricts the advancement and retract of the wire in the longitudinal direction.

## Description

### TECHNICAL FIELD

The present disclosure relates to an endoscopic treatment tool and an endoscopic treatment system. This application claims priority to U.S. Provisional Patent Application No. 63/375,987, filed in the United States on September 16, 2022, the contents of which are incorporated herein by reference.

### BACKGROUND ART

Endoscopic retrograde cholangio-pancreatography (ERCP) is used in a procedure in which a contrast agent, a guide wire, or various catheters are inserted from the duodenum into the bile duct to perform diagnosis within the bile duct, stone retrieval, drainage of strictures, and the like. In such procedures, a papillotome which allows insertion of an endoscope and the treatment tool channel of the endoscope is used.

The papillotome used in these procedures maintains a knife (a treatment part, an incision part) for incising the sphincter of nipple or the like in a state in which it can perform incisions by bending a distal end portion of a sheath. A papillotome described in Patent Document 1 has a rotation function that rotates a sheath around a central axis of the sheath, and can move a distal end portion equipped with a knife by rotating the sheath.

### Citation List

### Patent Document

Patent Document 1: Japanese Patent Publication No. 6856759

### SUMMARY

### Technical Problem

However, in the papillotome having a rotation function as described in Patent Document 1, it was difficult to maintain the distal end portion in a bent state while rotating the sheath and to keep the knife in a state capable of performing the incisions. For example, when the sheath is rotated, the distal end portion may not maintain the bent state.

In consideration of the above circumstances, an object of the present invention is to provide an endoscopic treatment tool that makes it easy to rotate a distal end portion provided with a treatment part while the distal end portion is maintained in a bent state, and an endoscopic treatment system that is equipped with the endoscopic treatment tool.

### Solution to Problem

In order to solve the above problems, the present invention proposes the following means.

An endoscopic treatment tool includes a sheath that is an elongated member having a lumen configured to extend in a longitudinal direction, a distal end treatment part provided at a distal end portion of the sheath, an operation part provided at a proximal end portion of the sheath, and a wire inserted through the lumen, mounted on the distal end treatment part, and supported by the operation part so as to advance and retract in the longitudinal direction, wherein the operation part has a locking mechanism that restricts the advancement and retract of the wire in the longitudinal direction.

### Advantageous Effects of Invention

The endoscopic treatment tool and the endoscopic treatment system of the present invention make it easy to rotate the distal end portion provided with the treatment part while the distal end portion is maintained in a bent state.

### BRIEF DESCRIPTION OF DRAWINGS

[FIG. 1] An overall view of an endoscopic treatment system according to a first embodiment.
[FIG. 2] A diagram showing a papillotome of the endoscopic treatment system.
[FIG. 3] A diagram showing the papillotome.
[FIG. 4] A cross-sectional view of a distal end portion of a sheath of the papillotome.
[FIG. 5] A front view of the distal end portion of the sheath when seen from the distal end side.
[FIG. 6] A cross-sectional view of an operation part of the papillotome.
[FIG. 7] A cross-sectional view of a proximal end portion of the sheath along line C1-C1 in FIG. 6.
[FIG. 8] An oblique view showing a first deformation part of a second main body.
[FIG. 9] A view showing the first deformation part before deformation and a first connection part.
[FIG. 10] A view showing the first deformation part after deformation and the first connection part.
[FIG. 11] A view showing the papillotome inserted into an endoscope.
[FIG. 12] A view showing an alignment step.
[FIG. 13] A view showing an incision step.
[FIG. 14] A diagram showing an operation part of a papillotome according to a second embodiment.
[FIG. 15] A diagram showing a first deformation part before deformation and a first connection part.
[FIG. 16] A diagram showing the first deformation part after deformation and the first connection part.
[FIG. 17] A diagram showing an operation part of a papillotome according to a third embodiment.
[FIG. 18] A diagram showing another aspect of an O-ring.
[FIG. 19] A diagram showing an operation part of a papillotome according to a fourth embodiment.
[FIG. 20] A diagram showing an operation part of a papillotome according to a fifth embodiment.
[FIG. 21] A diagram for describing an action of a lever of the operation part.
[FIG. 22] A diagram for describing an action of the lever.

### DESCRIPTION OF EMBODIMENTS

### (First embodiment)

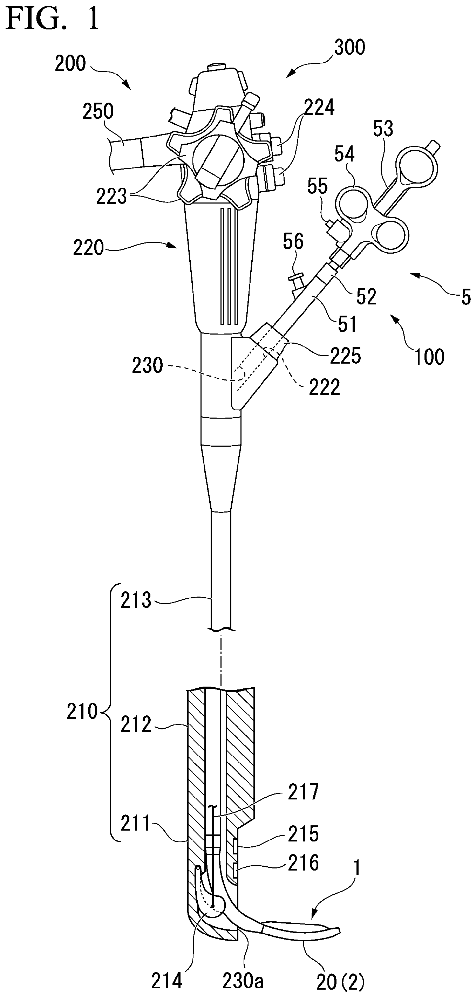

A first embodiment will be described with reference to FIG. 1 to FIG. 13. FIG. 1 is an overall view of an endoscopic treatment system 300 according to this embodiment.

### [Endoscopic treatment system 300]

The endoscopic treatment system 300 includes a papillotome 100 and an endoscope 200. The papillotome 100 is inserted into the endoscope 200 for use.

### [Endoscope 200]

The endoscope 200 is a side-viewing flexible endoscope, and includes a long insertion part 210, an operation part 220 provided at a proximal end portion of the insertion part 210, and a universal cord 250 that extends from the operation part 220. The endoscope 200 may be a direct-viewing flexible endoscope.

The insertion part 210 has a distal end hard part 211 provided at a distal end portion, a bending part 212 provided on the proximal end side of the distal end hard part 211, and a flexible tube part 213 provided on the proximal end side of the bending part 212. An imaging unit 216 including a light guide 215 and a CCD is provided on a side surface of the distal end hard part 211 in a state of being exposed to the outside.

A treatment tool channel 230 for inserting an endoscopic treatment tool such as a papillotome 100 is formed in the insertion part 210. A distal end portion 230a of the treatment tool channel 230 opens at a side surface of the distal end hard part 211. A proximal end portion of the treatment tool channel 230 extends to the operation part 220.

A lifting table 214 is provided on the distal end hard part 211 of the treatment tool channel 230. A proximal end portion of the lifting table 214 is supported by the distal end hard part 211 so as to be rotatable. A lifting table operating wire 217 fixed to a distal end portion of the lifting table 214 extends through the insertion part 210 to the proximal end side.

The bending part 212 is configured to be bendable in up-down and left-right directions. A distal end of an operating wire is fixed to the distal end side of the bending part 212. The operating wire extends through the insertion part 210 to the operation part 220.

A knob 223 for operating the operating wire and a switch 224 for operating the imaging unit 216, or the like are provided on the proximal end side of the operation part 220. A user can bend the bending part 212 in a desired direction by operating the knob 223.

A forceps port 222 that communicates with the treatment tool channel 230 is provided on the distal end side of the operation part 220. The user can insert the endoscopic treatment tool 100 from the forceps port 222. A forceps plug 225 is mounted in the forceps port 222 to prevent leakage of body fluids.

The universal cord 250 connects the endoscope 200 to an image control device. An image signal captured by the imaging unit 216 is transmitted to the image control device via the universal cord 250.

### [Papillotome 100]

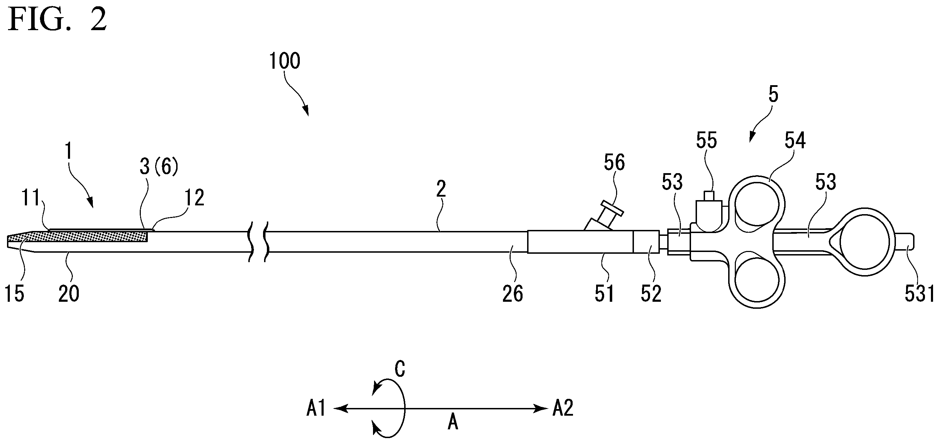

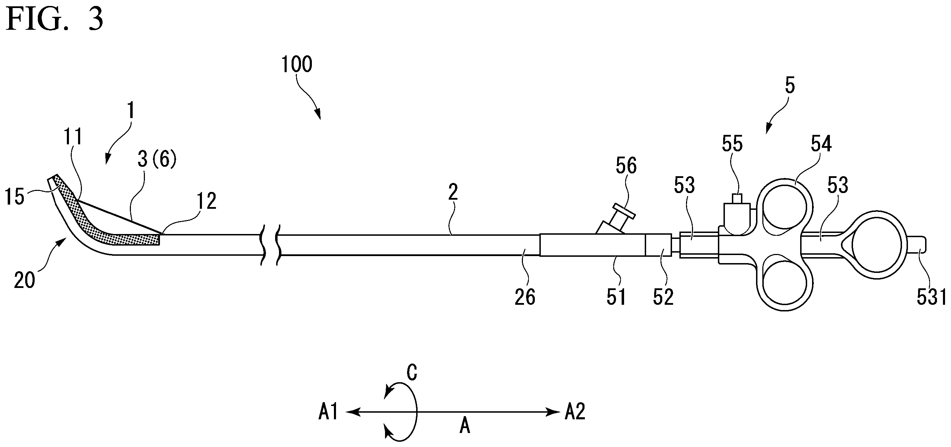

FIGS. 2 and 3 are diagrams showing the papillotome 100.

The papillotome (an endoscopic treatment tool, a high-frequency treatment tool) 100 includes a distal end treatment part 1, a sheath 2, a conductive wire 3, and an operation part 5. In the following description, in a longitudinal direction A of the papillotome 100, the side inserted into a patient's body is referred to as the "distal end side (the distal side) A1" and the operation part 5 side is referred to as the "proximal end side (the proximal side) A2."

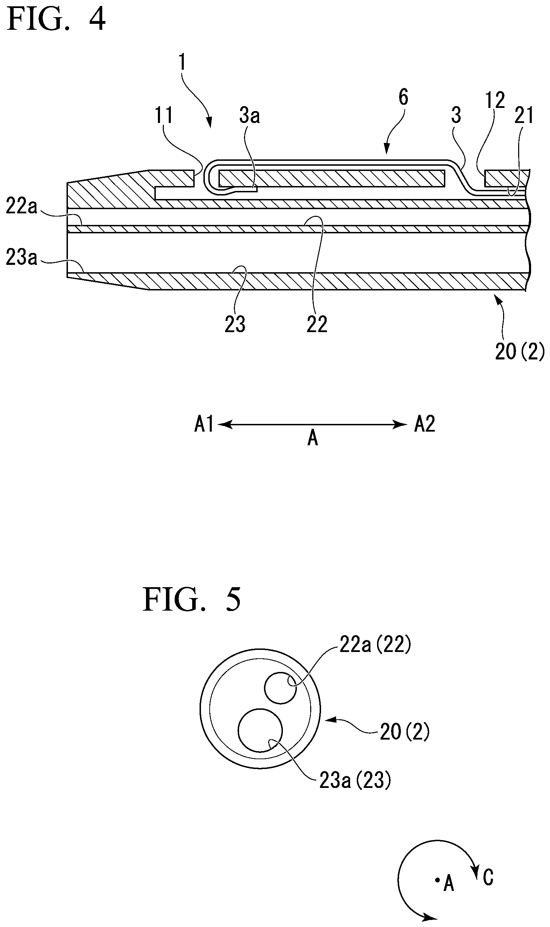

FIG. 4 is a cross-sectional view of a distal end portion 20 of the sheath 2.

The distal end treatment part 1 is provided on an outer circumferential surface of the distal end portion 20 of the sheath 2. The distal end treatment part 1 has a first through hole 11 and a second through hole 12 that pass through a knife lumen 21. The first through hole 11 and the second through hole 12 are arranged in the longitudinal direction A. The first through hole 11 is provided on the distal end side A1 with respect to the second through hole 12. The second through hole 12 is provided on the proximal end side A2 with respect to the first through hole 11. A marker 15 having a different color, pattern, or the like from other portions of the sheath 2 is attached on the distal end treatment part 1.

The marker 15 is formed in a line shape that extends in the longitudinal direction A, and is provided on a portion (a portion in which an incision part 6 described below is formed) as a part in the circumferential direction C in which the first through hole 11 and the second through hole 12 are provided. A distal end of the marker 15 is disposed at the distal end of the sheath 2, and a proximal end of the marker 15 is disposed in the vicinity of the second through hole 12. A width of the marker 15 in the circumferential direction C is equal to or less than half a length of an outer circumference of the sheath 2.

The sheath 2 is an elongated member having a lumen that extends in the longitudinal direction A. The sheath 2 has an outer diameter that can be inserted into the treatment tool channel 230. The sheath 2 is a soft sheath made of, for example, a resin.

FIG. 5 is a front view of the distal end portion 20 of the sheath 2 when seen from the distal end side A1.

The sheath 2 is a multi-lumen tube having a plurality of lumens. The plurality of lumens include the knife lumen 21 through which the conductive wire 3 is inserted, a contrast lumen 22 through which a contrast medium or the like is inserted, and a guide wire lumen 23 through which a guide wire is inserted.

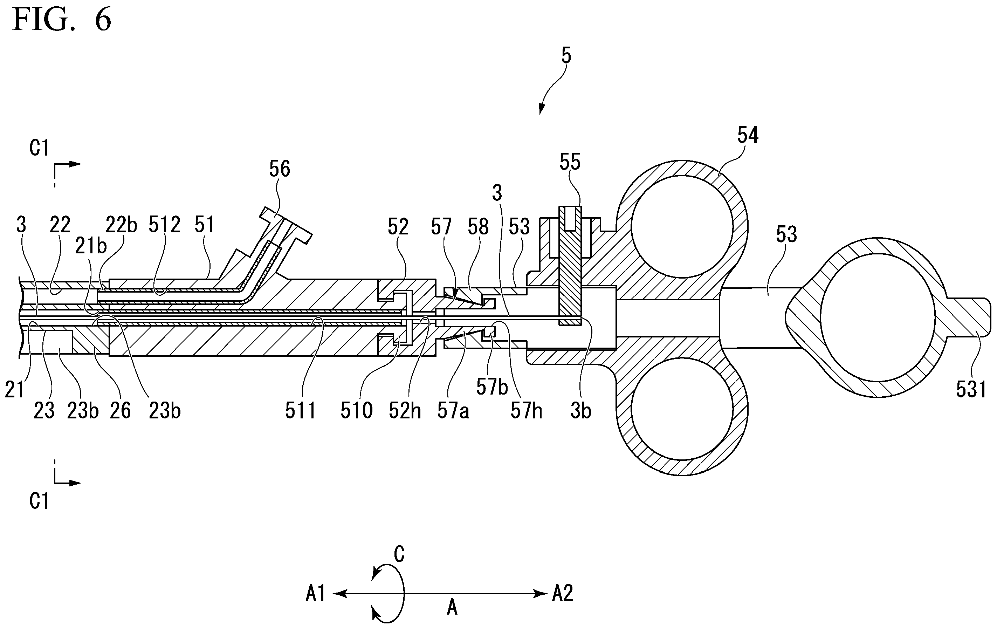

FIG. 6 is a cross-sectional view of the operation part 5.

The knife lumen 21 is a lumen through which the conductive wire 3 is inserted. The knife lumen 21 extends from a distal end opening (the first through hole 11 and the second through hole 12) provided in the distal end treatment part 1 to a proximal end opening 21b that communicates with the operation part 5.

The contrast lumen (a fluid supply lumen) 22 is a lumen through which a contrast medium, a water supply fluid, or an air supply fluid is inserted. The contrast lumen 22 extends from a distal end opening 22a formed at a distal end of the distal end portion 20 to a proximal end opening 22b that communicates with the operation part 5.

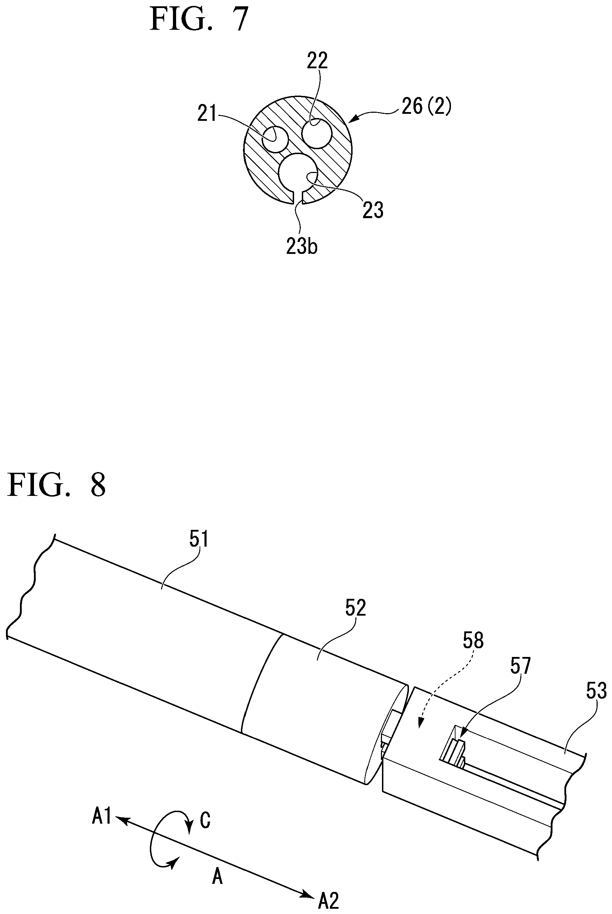

FIG. 7 is a cross-sectional view of a proximal end portion 26 of the sheath 2 along line C1-C1 in FIG. 6.

The guide wire lumen 23 is a lumen through which a guide wire is inserted. The guide wire lumen 23 extends from a distal end opening 23a formed at the distal end of the distal end portion 20 to a proximal end opening 23b provided on an outer circumferential surface of the proximal end portion 26 of the sheath 2.

The conductive wire 3 has a conductive core wire coated with an insulating film made of an appropriate synthetic resin. The conductive wire 3 is inserted through the knife lumen 21 of the sheath 2. The conductive wire 3 is supported by the operation part 5 so as to be capable of advancing and retracting in the longitudinal direction A and rotatable in the circumferential direction C.

A distal end portion of the conductive wire 3 protrudes to the outside of the sheath 2 from the knife lumen 21 at the second through hole 12, extends in the longitudinal direction A of the sheath 2 to the distal end side A1, and enters the knife lumen 21 from the first through hole 11 on the distal end side A1. A distal end 3a of the conductive wire 3 is fixed within the knife lumen 21. A portion of the conductive wire 3 that may be exposed to the outside of the sheath 2 from the first through hole 11 and the second through hole 12 is not coated with an insulating film, and the core wire is exposed to constitutes a wire-shaped incision part 6 that can incise tissue. The incision part 6 is an example of an electrode.

As shown in FIG. 6, a proximal end portion of the conductive wire 3 is mounted on a power supply connector 55 provided on a slider 54 of the operation part 5. When the conductive wire 3 is pulled toward the proximal end side A2, the distal end treatment part 1 is bent inward, and the incision part 6 is put in a taut state, as shown in FIG. 3. Since the portion with the marker 15 is bent inward, a surgeon can easily grasp a direction in which the distal end portion 20 of the sheath 2 is bent.

As shown in FIG. 6, the operation part 5 is provided at the proximal end of the sheath 2. The proximal end of the sheath 2 is mounted in the operation part 5 so as to be rotatable in the circumferential direction C. The operation part 5 includes a first main body 51, a second main body 52, a handle 53, the slider 54, and the power supply connector 55.

The first main body 51 is mounted on the proximal end of the sheath 2, and is formed in a shape extending in the longitudinal direction A. The first main body 51 has a proximal end convex portion 510, a first tube 511, a second tube 512, and a contrast mouthpiece 56.

The first tube 511 passes through the inside of the first main body 51, and a distal end thereof communicates with the knife lumen 21. The conductive wire 3 is inserted through the first tube 511.

The second tube 512 is inserted through the inside of the first main body 51, and a distal end thereof communicates with the contrast lumen 22. A proximal end of the second tube 512 communicates with an opening of the contrast mouthpiece 56. A fluid such as a contrast medium injected through the contrast mouthpiece 56 passes through the second tube 512 and the contrast lumen 22 and is discharged from a distal end opening 22a formed at the distal end of the distal end portion 20.

FIG. 8 is a perspective view showing a first deformation part 57 of the second main body 52.

The second main body 52 is mounted on the proximal end convex portion 510 of the first main body 51. The second main body 52 is mounted on the first main body 51 so as to be rotatable in the circumferential direction C. The second main body 52 has a through hole 52h passing therethrough in the longitudinal direction A. The conductive wire 3 is inserted through the through hole 52h. The first deformation part 57 connected to the handle 53 is provided at the proximal end of the second main body 52.

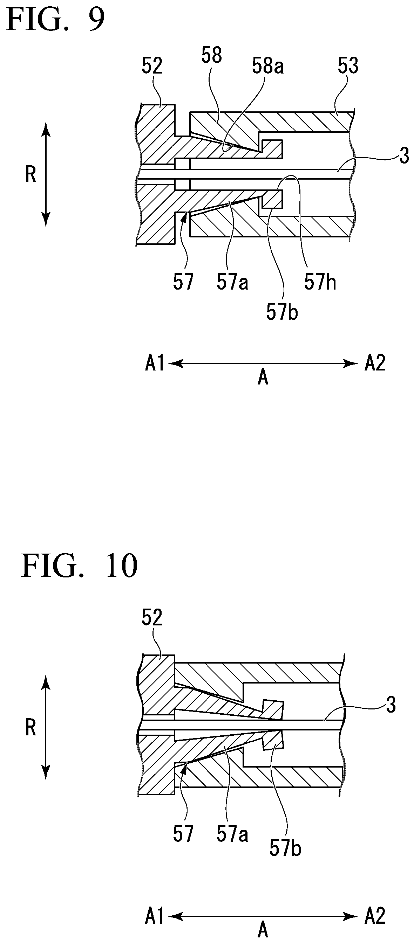

FIG. 9 shows the first deformation part 57 before deformation and a first connection part 58.

The first deformation part 57 is an elastically deformable member, and is formed into a substantially wedge-shaped convex portion. The first deformation part 57 has a tapered portion 57a, a stopper 57b, and a through hole 57h. The tapered portion 57a is formed to have a quadrangular column shape of which top and bottom surfaces are identical trapezoids. A width of the tapered portion 57a decreases from the distal end side A1 to the proximal end side A2. The stopper 57b is provided at a proximal end of the tapered portion 57a and prevents the first deformation part 57 from being separated from the handle 53. The through hole 57h passes through the first deformation part 57 in the longitudinal direction A. The conductive wire 3 is inserted through the through hole 57h.

As shown in FIG. 8, the handle 53 is connected to the second main body 52 so as not to be rotatable in the circumferential direction C with respect to the second main body 52. When the handle 53 is rotated in the circumferential direction C, the second main body 52 rotates together with the handle 53 with respect to the first main body 51. The first connection part 58 that is connected to the first deformation part 57 of the second main body 52 is provided at a distal end of the handle 53.

The first connection part 58 is formed into a substantially wedge-shaped concave portion, and the first deformation part 57 is disposed inward in the radial direction R. An inner surface 58a of the first connection part 58 is formed in a mortar shape that is recessed into a quadrangular column shape of which the top and bottom surfaces are identical trapezoids, and a width thereof decreases from the distal end side A1 toward the proximal end side A2.

FIG. 10 is a diagram showing the first deformation part 57 after deformation and the first connection part 58.

By bringing the second main body 52 and the handle 53 closer to each other, the first deformation part 57 engages with the inner surface 58a of the first connection part 58, and is deformed inward in the radial direction R. As a result, the first deformation part 57 comes into contact with the conductive wire 3 from the outside in the radial direction R, and restricts advancement and retract of the conductive wire 3 in the longitudinal direction A.

That is, due to the engagement with the first connection part 58, the first deformation part 57 functions as a locking mechanism that restricts the advancement and retract of the conductive wire 3 in the longitudinal direction A. Here, the "locking mechanism" refers to a mechanism that can restrict the advancement and retract of the conductive wire 3 in the longitudinal direction A without restricting rotation of the conductive wire 3 in the circumferential direction C. The locking mechanism may restrict the rotation of the conductive wire 3 in the circumferential direction C.

A knob 531 is provided at a proximal end of the handle 53. A user can easily rotate the handle 53 in the circumferential direction C by gripping the knob 531 and rotating it in the circumferential direction C.

The slider 54 is mounted on the handle 53 so as to advance and retract in the longitudinal direction A but not to be rotatable in the circumferential direction C.

The power supply connector 55 is fixed to the slider 54. A proximal end 3b of the conductive wire 3 is connected to the power supply connector 55. The power supply connector 55 can be connected to a high-frequency power supply device (not shown), and is connected to the conductive wire 3. The power supply connector 55 may supply high-frequency current supplied from the high-frequency power supply device to the conductive wire 3 and the incision part 6.

By rotating the handle 53 in the circumferential direction C with respect to the first main body 51, the second main body 52, the slider 54, and the power supply connector 55 rotate together. The conductive wire 3 rotates in the circumferential direction C with respect to the first main body 51. As a result, the distal end treatment part 1 provided at the distal end portion 20 of the sheath 2 rotates. That is, the conductive wire 3 also serves as a torque transmission member that transmits rotational torque in the circumferential direction C.

### [Operation of endoscopic treatment system 300]

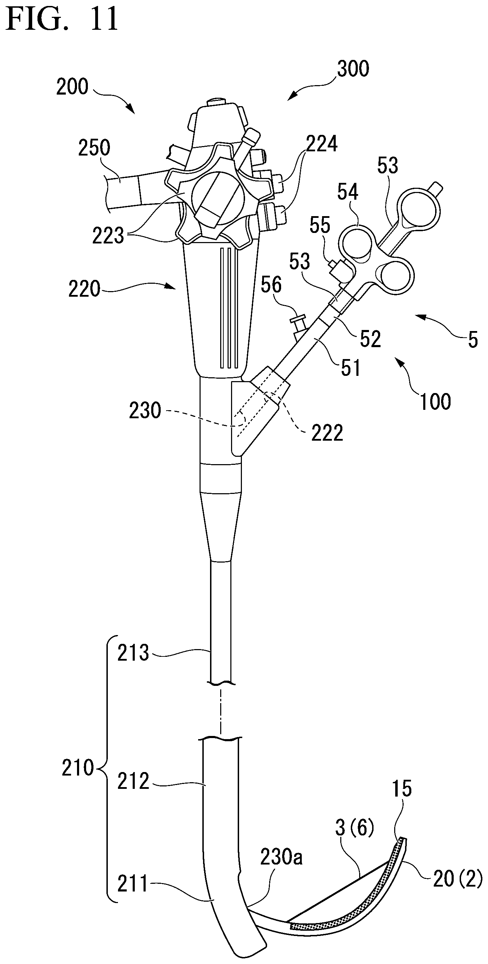

Next, an operation and action of the endoscopic treatment system 300 according to this embodiment will be described with reference to FIGS. 11 to 13. Specifically, a procedure for treating a bile duct BD by endoscopic retrograde cholangio-pancreatography (ERCP) will be described.

### <Step S1: Endoscope insertion step>

In Step S1, the surgeon inserts the insertion part 210 of the endoscope 200 into a patient's lumen through a natural opening such as a mouth. If necessary, the surgeon operates the operation part 220 to bend the bending part 212. The surgeon inserts the distal end hard part 211 of the endoscope 200 up to the vicinity of a duodenal papilla.

### <Step S2: Endoscope positioning step>

In Step S2, the surgeon adjusts a position of the distal end hard part 211 of the endoscope 200 so that the duodenal papilla is within an imaging range of the imaging unit 216 of the endoscope 200. The surgeon adjusts the position of the distal end hard part 211 by appropriately combining the advancement and retract, bending, twisting, and other operations of the endoscope 200.

### <Step S3: Papillotome positioning step>

FIG. 11 shows the papillotome 100 inserted into the endoscope 200.

Next, in Step S3, the surgeon inserts the papillotome 100 into the treatment tool channel 230 from the forceps port (the proximal end opening) 222 of the endoscope 200. The surgeon protrudes the distal end portion 20 of the sheath 2 from the distal end opening 230a.

The surgeon moves the slider 54 toward the proximal end side A2 with respect to the handle 53 to pull the conductive wire 3 and to bend the distal end portion 20 of the sheath 2. As a result, as shown in FIG. 11, the incision part 6 which is a part of the conductive wire 3 is put in a taut state. Next, the surgeon brings the main body (the first main body 51 and the second main body 52) closer to the handle 53. As a result, the first deformation part 57 is deformed inward in the radial direction R to come into contact with the conductive wire 3, thereby restricting the advancement and retract of the conductive wire 3. The surgeon may move the distal end treatment part 1 by rotating the sheath 2 in the circumferential direction C while the distal end portion 20 of the sheath 2 is maintained in a bent state. The surgeon may rotate the entire papillotome 100 in the circumferential direction C to move the distal end treatment part 1.

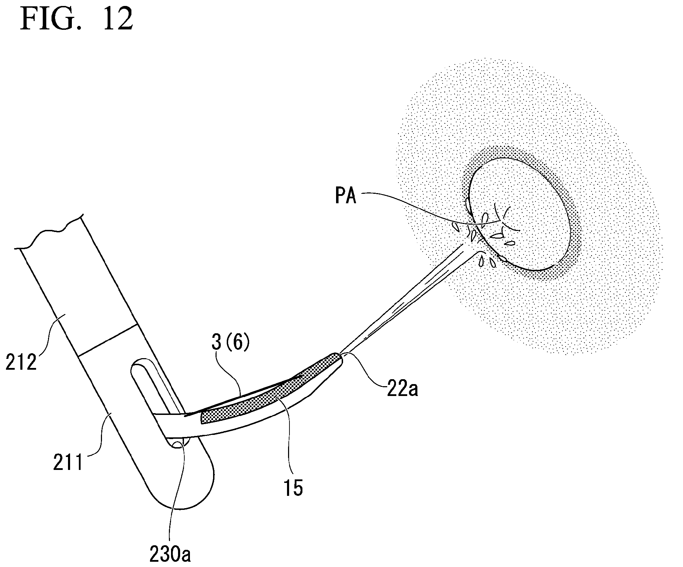

FIG. 12 is a diagram showing a positioning step.

The surgeon may discharge the fluid from the distal end opening 22a via the contrast lumen (the fluid supply lumen) 22 to confirm whether the distal end portion 20 of the sheath 2 is facing a papilla PA. When the surgeon may spray a fluid onto the papilla PA from the distal end opening 22a, the surgeon can confirm that the distal end portion 20 of the sheath 2 has been disposed in the vicinity of the papilla PA.

### <Step S4: Incision step>

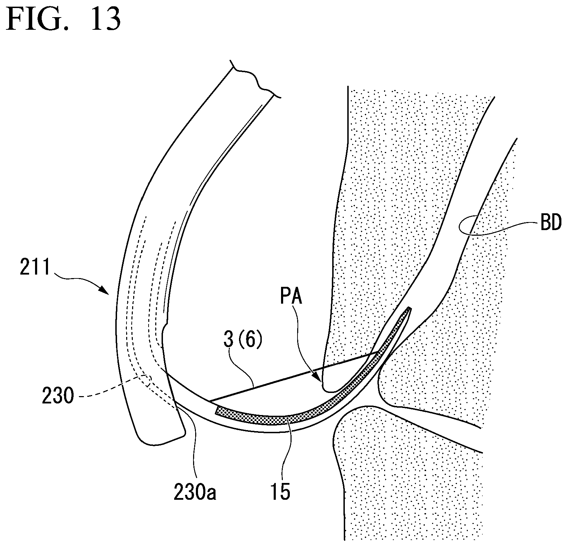

FIG. 13 shows an incision step.

In Step S4, the surgeon supplies a high-frequency current to the incision part 6 from the power supply connector 55, and operates the operation part 5 of the papillotome 100 to incise a part of the papilla PA with the incision part 6.

### <Step S5: Contrast medium injection step>

In Step S5, the surgeon injects a contrast medium into a bile duct BD from the papillotome 100. The surgeon then performs X-ray photography to obtain an X-ray image showing the bile duct BD and the like.

### <Step S6: Guide wire insertion step>

In Step S6, the surgeon inserts a guide wire into the guide wire lumen 23 of the papillotome 100, causes the guide wire to protrude from the distal end opening 23a, and inserts the guide wire into the bile duct BD. Next, the surgeon removes the papillotome 100 while the guide wire is left in the bile duct BD. As a result, only the guide wire may be placed in the bile duct BD, and an access route from the duodenum to the bile duct BD is formed.

After the access route is formed, the surgeon can perform various treatments by introducing distal end portions of various treatment tools along the guide wire into the bile duct BD via the access route.

According to the papillotome 100 of this embodiment, while the distal end portion 20 of the sheath 2 provided with the distal end treatment part 1 is maintained in the bent state, the sheath 2 can be rotated in the circumferential direction C to easily move the distal end portion 20. Therefore, the surgeon may perform an operation of rotating the sheath 2 in the circumferential direction C by himself while the distal end portion 20 of the sheath 2 is maintained in the bent state.

The first embodiment has been described above in detail with reference to the drawings. However, the specific configuration is not limited to this embodiment, and design changes and the like that do not deviate from the gist of the present disclosure are also included. Furthermore, the components shown in the above-described embodiment and the modified examples shown below can be combined as appropriate.

### (Second embodiment)

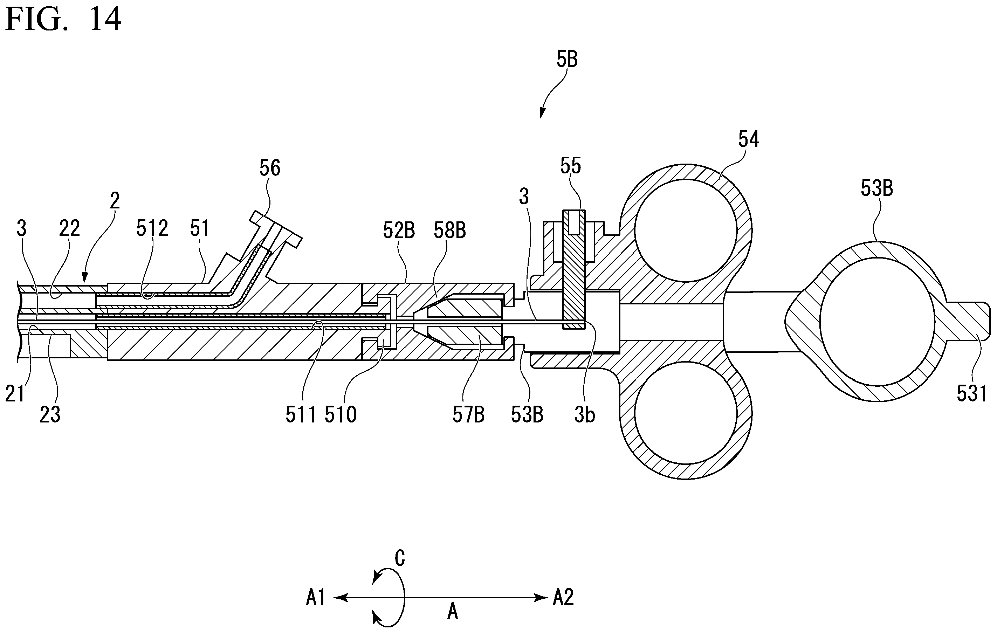

A papillotome 100B according to a second embodiment will be described with reference to FIGS. 14 to 16. In the following description, components common to those already described will be given the same reference numerals, and duplicated description will be omitted.

FIG. 14 is a diagram showing an operation part 5B of the papillotome 100B.

The papillotome (the endoscopic treatment tool, the high-frequency treatment tool) 100B is used together with an endoscope 200 as an endoscopic treatment system, similar to the papillotome 100 of the first embodiment. The papillotome 100B includes a distal end treatment part 1, a sheath 2, a conductive wire 3, and the operation part 5B.

The operation part 5B includes a first main body 51, a second main body 52B, a handle 53B, a slider 54, and a power supply connector 55.

The second main body 52B is mounted on a proximal end convex portion 510 of the first main body 51. The second main body 52B is mounted on the first main body 51 so as to be rotatable in the circumferential direction C. The second main body 52B has a through hole 52h that passes therethrough in the longitudinal direction A. The conductive wire 3 is inserted through the through hole 52h. In addition, a second connection part 58B is provided at a proximal end of the second main body 52.

The second connection part 58B is formed in a substantially wedge-shaped concave portion, and a second deformation part 57B is disposed inward in the radial direction R. An inner surface 58Ba of the second connection part 58B is formed in mortar shape that is recessed into a quadrangular column shape of which top and bottom surfaces are identical trapezoids, and a width thereof increases from the distal end side A1 toward the proximal end side A2.

The handle 53B is connected to the second main body 52B so as not to be rotatable in the circumferential direction C with respect to the second main body 52B. When the handle 53B is rotated in the circumferential direction C, the second main body 52B rotates with respect to the first main body 51 together with the handle 53B. The second deformation part 57B that is connected to the second connection part 58B of the second main body 52B is provided at a distal end of the handle 53B.

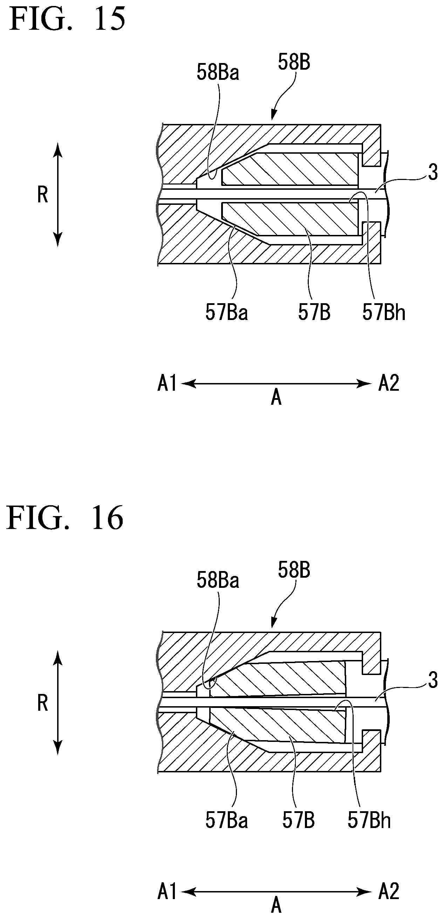

FIG. 15 shows the second deformation part 57B before deformation and the second connection part 58B.

The second deformation part 57B is an elastically deformable member, and is formed into a substantially wedge-shaped convex portion. The second deformation part 57B has a tapered portion 57Ba and a through hole 57Bh. The tapered portion 57Ba is formed in a quadrangular column shape of which top and bottom surfaces are identical trapezoids. A width of the tapered portion 57Ba increases from the distal end side A1 to the proximal end side A2. The through hole 57Bh passes through the second deformation part 57B in the longitudinal direction A. The conductive wire 3 is inserted through the through hole 57Bh.

FIG. 16 is a diagram showing the second deformation part 57B after deformation and the second connection part 58B.

When the second main body 52B and the handle 53B are brought closer to each other, the second deformation part 57B is deformed inward in the radial direction R by engaging with the inner surface 58Ba of the second connection part 58B. As a result, the second deformation part 57B comes into contact with the conductive wire 3 from the outside in the radial direction R, and restricts the advancement and retract of the conductive wire 3 in the longitudinal direction A.

According to the papillotome 100B of this embodiment, the distal end portion 20 of the sheath 2 provided with the distal end treatment part 1 can be easily moved by rotating the sheath 2 in the circumferential direction C while the distal end portion 20 is maintained in a bent state. Therefore, the surgeon can perform the operation of rotating the sheath 2 in the circumferential direction C by himself while the distal end portion 20 of the sheath 2 is maintained in the bent state.

The second embodiment has been described above in detail with reference to the drawings. However, the specific configuration is not limited to this embodiment, and design changes and the like that do not deviate from the gist of the present disclosure are also included. Furthermore, the components shown in the above-described embodiment and the modified examples shown below can be combined as appropriate.

### (Third embodiment)

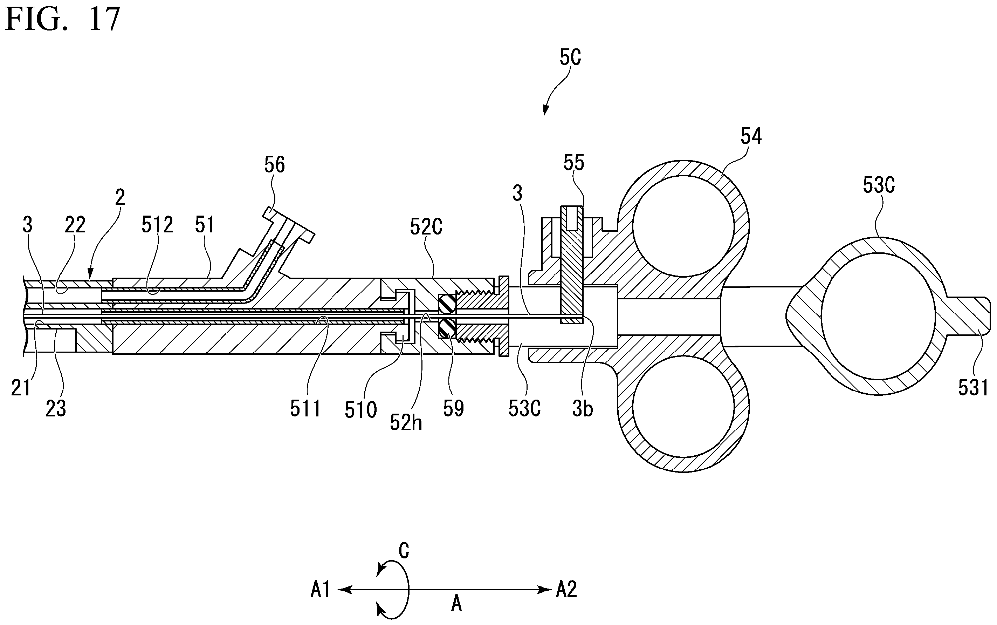

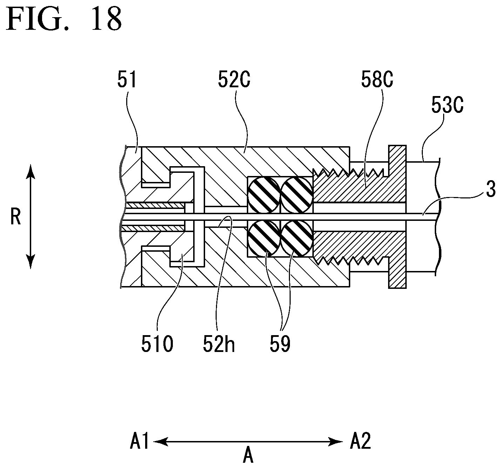

A papillotome 100C according to a third embodiment will be described with reference to FIGS. 17 and 18. In the following description, components common to those already described will be given the same reference numerals, and duplicated description will be omitted.

FIG. 17 is a diagram showing an operation part 5C of the papillotome 100C.

The papillotome (the endoscopic treatment tool, the high-frequency treatment tool) 100C is used together with an endoscope 200 as an endoscopic treatment system, similar to the papillotome 100 of the first embodiment. The papillotome 100C includes a distal end treatment part 1, a sheath 2, a conductive wire 3, and the operation part 5C.

The operation part 5C includes a first main body 51, a second main body 52C, a handle 53C, a slider 54, and a power supply connector 55.

The second main body 52C is mounted on a proximal end of the first main body 51. The second main body 52C has a through hole 52h that passes therethrough in the longitudinal direction A. The conductive wire 3 is inserted through the through hole 52h.

The through hole 52h of the second main body 52C accommodates an O-ring (a ring member) 59. The conductive wire 3 is inserted through the O-ring 59 and is in contact with the O-ring 59. Therefore, compared to the first embodiment, sliding resistance of the conductive wire 3 in the longitudinal direction A is high. Therefore, even when the surgeon releases his/her hand from the slider 54, the conductive wire 3 is less likely to advance in the longitudinal direction A, and the distal end portion 20 of the sheath 2 is more likely to maintain the bent state.

FIG. 18 is a diagram showing another aspect of the O-ring 59.

The second main body 52C may accommodate a plurality of O-rings 59 as shown in FIG. 18. This further increases the sliding resistance of the conductive wire 3 in the longitudinal direction A.

The handle 53C is connected to the second main body 52C so as not to be rotatable in the circumferential direction C with respect to the second main body 52C and not to advance and retract in the longitudinal direction A. When the handle 53C is rotated in the circumferential direction C, the second main body 52C rotates together with the handle 53C with respect to the first main body 51.

According to the papillotome 100C of this embodiment, the distal end portion 20 of the sheath 2 provided with the distal end treatment part 1 can be easily moved by rotating the sheath 2 in the circumferential direction C while the distal end portion 20 is maintained in a bent state. The surgeon can maintain the bent state of the distal end portion 20 without performing any special operation. Therefore, the surgeon can perform an operation of rotating the sheath 2 in the circumferential direction C by himself while the distal end portion 20 of the sheath 2 is maintained in the bent state.

The third embodiment has been described above in detail with reference to the drawings. However, the specific configuration is not limited to this embodiment, and design changes and the like that do not deviate from the gist of the present disclosure are also included. Furthermore, the components shown in the above-described embodiment and the modified examples shown below can be combined as appropriate.

### (Fourth embodiment)

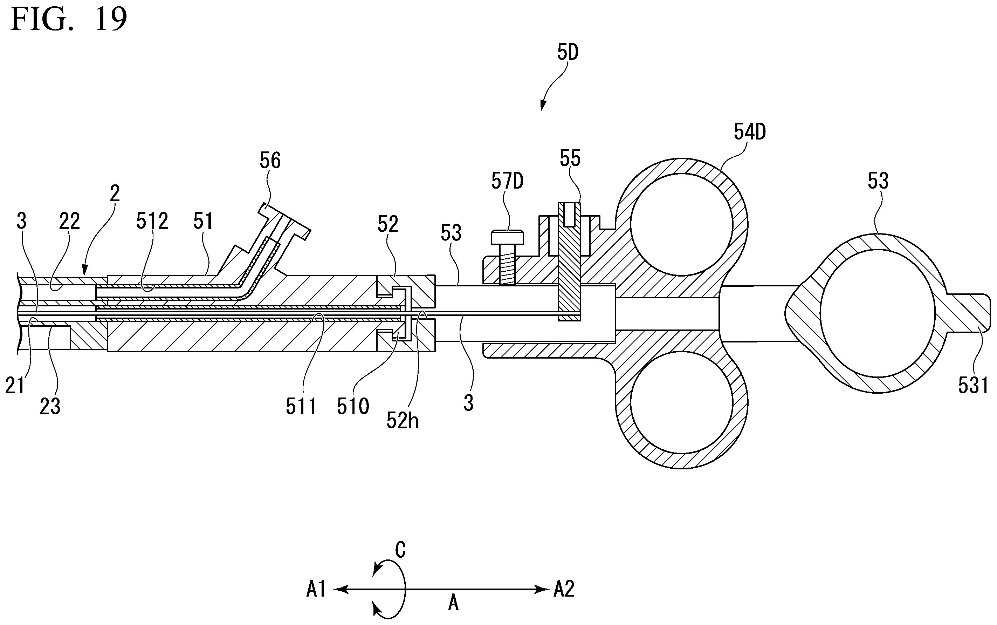

A papillotome 100D according to a fourth embodiment will be described with reference to FIG. 19. In the following description, components common to those already described will be given the same reference numerals, and duplicated description will be omitted.

FIG. 19 is a diagram showing an operation part 5D of the papillotome 100D.

The papillotome (the endoscopic treatment tool, the high-frequency treatment tool) 100D is used as an endoscopic treatment system together with the endoscope 200, similar to the papillotome 100 of the first embodiment. The papillotome 100D includes a distal end treatment part 1, a sheath 2, a conductive wire 3, and the operation part 5D.

The operation part 5D includes a first main body 51, a second main body 52, a handle 53, a slider 54D, and a power supply connector 55.

The slider 54D is mounted on the handle 53 so as to advance and retract in the longitudinal direction A and so as not to be rotatable in the circumferential direction C. The slider 54D has a screw 57D.

The screw (a first pressing member) 57D can fix a position of the slider 54D with respect to the handle 53.

The surgeon moves the slider 54D toward the proximal end side A2 with respect to the handle 53 to pull the conductive wire 3 and to bend the distal end portion 20 of the sheath 2. The surgeon fixes the position of the slider 54D with respect to the handle 53 by tightening the screw 57D while the distal end portion 20 of the sheath 2 is in a bent state. As a result, even when the surgeon releases his/her hand from the slider 54D, the conductive wire 3 does not advance in the longitudinal direction A, and the distal end portion 20 of the sheath 2 can be maintained in the bent state.

According to the papillotome 100D of this embodiment, the distal end portion 20 of the sheath 2 provided with the distal end treatment part 1 can be easily moved by rotating the sheath 2 in the circumferential direction C while the distal end portion 20 is maintained in the bent state.

The fourth embodiment has been described above in detail with reference to the drawings. However, the specific configuration is not limited to this embodiment, and design modifications and the like that do not deviate from the gist of the present disclosure are also included. Furthermore, the components shown in the above-described embodiment and the modified examples shown below can be combined as appropriate.

### (Fifth embodiment)

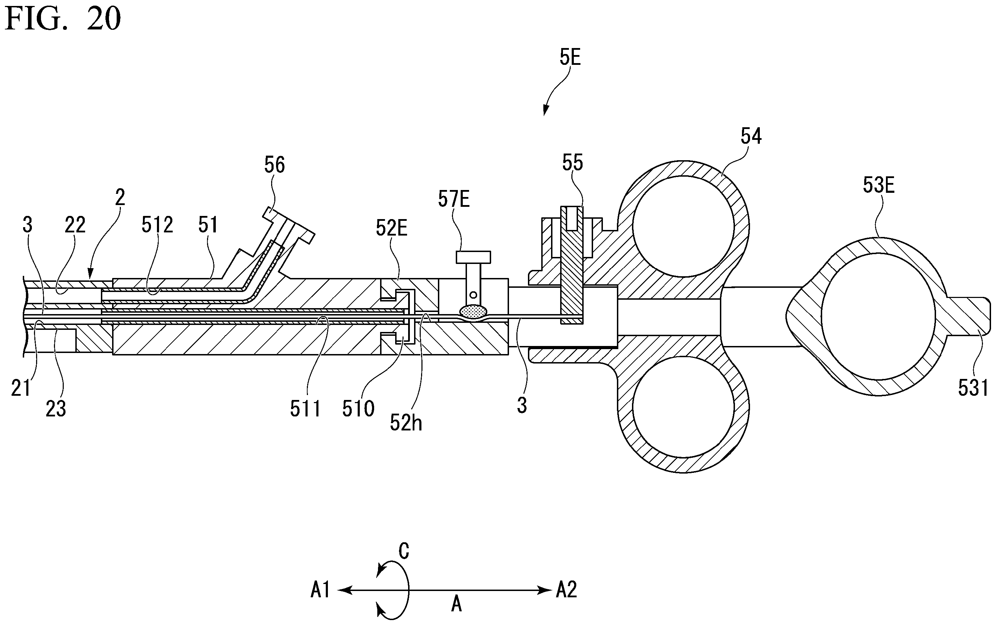

A papillotome 100E according to a fifth embodiment will be described with reference to FIG. 20 to FIG. 22. In the following description, components common to those already described will be given by the same reference numerals, and duplicated description will be omitted.

FIG. 20 is a diagram showing an operation part 5E of the papillotome 100E.

The papillotome (the endoscopic treatment tool, the high-frequency treatment tool) 100E is used as an endoscopic treatment system together with the endoscope 200, similar to the papillotome 100 of the first embodiment. The papillotome 100E includes a distal end treatment part 1, a sheath 2, a conductive wire 3, and the operation part 5E.

The operation part 5E includes a first main body 51, a second main body 52E, a handle 53E, a slider 54, and a power supply connector 55.

The second main body 52E is mounted on a proximal end of the first main body 51. A through hole 52h that passes therethrough in the longitudinal direction A is formed in the second main body 52E. The conductive wire 3 is inserted through the through hole 52h. A lever 57E is provided at a proximal end of the second main body 52E.

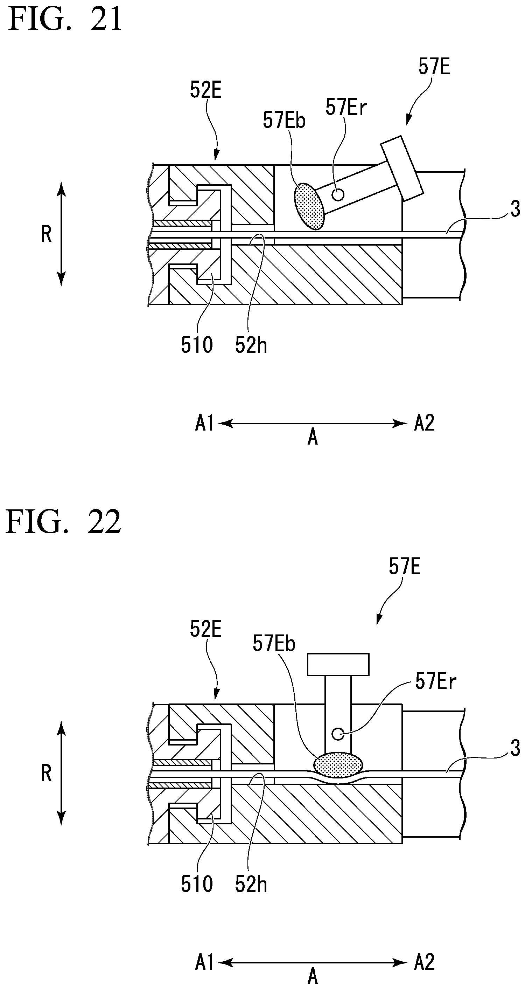

FIGS. 21 and 22 are diagrams describing a function of the lever 57E.

A lever (a second pressing member) 57E is mounted on the second main body 52E so as to be rotatable around a rotation shaft 57Er. The lever 57E has a pressing portion 57Eb capable of coming into contact with the conductive wire 3. By rotating the lever 57E to arrange the pressing portion 57Eb in a position at which it presses the conductive wire 3, a position of the conductive wire 3 with respect to the second main body 52 can be fixed.

The surgeon moves the slider 54 toward the proximal end side A2 with respect to the handle 53 to pull the conductive wire 3 and to bend the distal end portion 20 of the sheath 2. The surgeon fixes the position of the conductive wire 3 with respect to the second main body 52 by arranging the pressing portion 57Eb in the position at which it presses the conductive wire 3 while the distal end portion 20 of the sheath 2 is in a bent state. As a result, even when the surgeon releases his/her hand from the slider 54, the conductive wire 3 does not advance in the longitudinal direction A, and the distal end portion 20 of the sheath 2 can be maintained in the bent state.

The handle 53E is connected to the second main body 52E so as not to be rotatable in the circumferential direction C with respect to the second main body 52E and not to advance and retract in the longitudinal direction A. When the handle 53E is rotated in the circumferential direction C, the second main body 52E rotates with respect to the first main body 51 together with the handle 53E.

According to the papillotome 100E of this embodiment, the distal end portion 20 of the sheath 2 provided with the distal end treatment part 1 can be easily moved by rotating the sheath 2 in the circumferential direction C while the distal end portion 20 is maintained in the bent state.

The fifth embodiment has been described above in detail with reference to the drawings. However, the specific configuration is not limited to this embodiment, and design modifications and the like that do not deviate from the gist of the present disclosure are also included. Furthermore, the components shown in the above-described embodiment and the modified examples shown below can be combined as appropriate.

### INDUSTRIAL APPLICABILITY

The present disclosure can be applied to an endoscopic treatment tool having a rotation function.

### REFERENCE SIGNS LIST

300 Endoscopic treatment system
200 Endoscope
100, 100B, 100C, 100D, 100E Papillotome (endoscopic treatment tool, high-frequency treatment tool)
1 Distal end treatment part
11 First through hole
12 Second through hole
15 Marker
2 Sheath
20 Distal end portion
21 Knife lumen
21b Proximal end opening
22 Contrast lumen (fluid supply lumen)
22a Distal end opening
22b Proximal end opening
23 Guide wire lumen
23a Distal end opening
23b Proximal end opening
26 Proximal end portion
3 Conductive wire
5, 5B, 5C, 5D, 5E Operation part
51 First main body
511 First tube
512 Second tube
52, 52B, 52C, 52E Second main body
52h Through hole
53, 53B, 53C, 53E Handle
531 Knob
54, 54D Slider
55 Power supply connector
56 Contrast mouthpiece
57 First deformation part
57B Second deformation part
57Bh Through hole
57a, 57Ba Tapered portion
57D Screw (first pressing member)
57E Lever (Second pressing member)
57Eb Pressing portion
57Er Rotation shaft
57h Through hole
58 First connection part
58a Inner surface
58B Second connection part
58Ba Inner surface
59 O-ring (ring member)
6 Incision part

## Claims

1. An endoscopic treatment tool comprising:
a sheath that is an elongated member having a lumen, and extending in a longitudinal direction;
a treatment part disposed distally relative to the sheath;
an operation part disposed proximally relative to the sheath; and
a wire inserted through the lumen, mounted on the treatment part, and supported by the operation part so as to advance and retract in the longitudinal direction,
wherein the operation part has a locking mechanism configured to restrict the advancement and retract of the wire in the longitudinal direction.

2. The endoscopic treatment tool according to claim 1, wherein the wire is supported by the operation part so as to be rotatable in a circumferential direction.

3. The endoscopic treatment tool according to claim 1, wherein
the treatment part has a first through hole and a second through hole that communicate with the lumen, and
the wire is inserted through the first through hole and the second through hole and exposed to an outside of the sheath.

4. The endoscopic treatment tool according to claim 2, wherein the sheath is mounted on the operation part so as to be rotatable in the circumferential direction, and
the sheath is configured to rotate in the circumferential direction in response to an rotation of the wire in the circumferential direction.

5. The endoscopic treatment tool according to claim 1, wherein the operation part comprises:
a main body mounted on a proximal end of the sheath;
a handle connected to the main body so as to be rotatable in the circumferential direction; and
a slider connected to the handle so as to advance and retract in the longitudinal direction,
wherein the wire is connected to a connector provided on the slider.

6. The endoscopic treatment tool according to claim 5, wherein
the locking mechanism comprises a first deformation part provided on the main body, and
the first deformation part is configured to be deformed inward in a radial direction when the main body and the handle move closer to each other, and
the first deformation part is configured to contact with the wire to restrict the advancement and retract of the wire.

7. The endoscopic treatment tool according to claim 5, wherein
the locking mechanism comprises a second deformation part provided on the handle, and
the second deformation part is configured to be deformed inward in a radial direction when the main body and the handle move closer to each other, and
the second deformation part is configured to contact with the wire to restrict the advancement and retract of the wire.

8. The endoscopic treatment tool according to claim 5, wherein
the locking mechanism comprises a ring member configured to contact with the wire provided on the main body.

9. The endoscopic treatment tool according to claim 5, wherein
the locking mechanism comprises a first pressing member provided on the slider, and
the first pressing member is configured to fix a position of the slider with respect to the handle.

10. The endoscopic treatment tool according to claim 5, wherein
the locking mechanism comprises a second pressing member provided on the main body, and
the second pressing member is configured to fix a position of the wire with respect to the main body.

11. The endoscopic treatment tool according to claim 3, wherein
a marker is provided at a portion of the sheath in a circumferential direction in which the first through hole and the second through hole are provided.

12. An endoscopic treatment system comprising:
the endoscopic treatment tool according to claim 1; and
an endoscope.
